(19) Europäisches Patentamt

European Patent Office

Office européen des brevets

(11) **EP 0 541 671 B1**

(12) **EUROPEAN PATENT SPECIFICATION**

(45) Date of publication and mention of the grant of the patent:
**12.04.2000 Bulletin 2000/15**

(21) Application number: **91914382.6**

(22) Date of filing: **30.07.1991**

(51) Int. Cl.[7]: **A61B 5/03**, G01L 9/00

(86) International application number:
**PCT/US91/05394**

(87) International publication number:
**WO 92/02174 (20.02.1992 Gazette 1992/05)**

(54) **METHOD AND APPARATUS FOR MEASURING INTRACRANIAL PRESSURE**

VERFAHREN UND GERÄT ZUR MESSUNG DES INTRAKRANIALEN DRUCKES

PROCEDE ET APPAREIL DE MESURE DE LA PRESSION INTRACRANIENNE

(84) Designated Contracting States:
**AT BE CH DE DK ES FR GB GR IT LI LU NL SE**

(30) Priority: **31.07.1990 US 560162**

(43) Date of publication of application:
**19.05.1993 Bulletin 1993/20**

(73) Proprietor: **MEDYS, INC.**
**Chester, New Hampshire 03036 (US)**

(72) Inventor: **MICK, Edwin C.**
**Chester, NH 03036 (US)**

(74) Representative:
**Watts, Peter Graham et al**
**Anthony Cundy & Co.,**
**1 Olton Bridge,**
**245 Warwick Road**
**Solihull, West Midlands B92 7AH (GB)**

(56) References cited:
• **Journal of Clinical Engineering, Vol. 7, issued 1982, J.L. SEMMLOW and R. FISHER, "A Noninvasive Approach to Intracranial Monitoring", see pages 73-78.**
• **Core of the Professional Voice, Invited Paper, Sixteenth Symposium, Lincoln Center, New York, June 1987, R. COLEMAN, "Comparison of Microphone and Neck Mounted Accelerometer Monitoring the Performing Voice", see page 131.**
• **CYRIL M.HARRIS: 'Shock and vibration handbook', 1988, MCGRAW-HILL BOOK COMPANY, 3RD. EDITION, NEW YORK AO. Chapter 12, pages 12-1 to 12-27**

**Description**

## BACKGROUND OF THE INVENTION

FIELD OF THE INVENTION

The present invention relates generally to the measurement of intracranial pressure. More specifically, this invention relates to the provision of an improved method and apparatus for the non-invasive measurement of changes in intracranial pressure.

BRIEF DESCRIPTION OF THE PRIOR ART

Measurement of intracranial pressure (ICP) has become a routine neurosurgical procedure used in monitoring patients with conditions such as head injury, intracranial infection, hemorrhage, and hydrocephalus.

Generally when ICP reaches 2660 Pa (20 mm Hg) it becomes a concern and when it reaches 3325 Pa (25 mm Hg) or more than 2 minutes it is considered life threatening. Normal ICP is considered to be 0-532 Pa (0-4 mm Hg).

There are a number of prior references on the measurement of fluid pressures within the body cavity. The applicable work known to applicant is set forth in the following references:

Murr, U.S. Patent No. 3,853,117 - fluid pressure within a body cavity is measured with the use of a sonic transponder implanted inside the body cavity, e.g., the cranium. A sound signal is sent to the transponder to emit a resonance signal which is received at an external detector. The method is based on the principle that the skull and the cranial fluid are good sound conductors and that there are no intervening structures to attenuate the input or output signals. The implanted transponder has a diaphragm which serves as a mechanically resonant structure. This technique has major drawbacks, however. It involves invasive methods which introduce significant risks due to the possibility of cerebral infections. Furthermore, the measurement of a sound signal by an external detector affects deleteriously the measured sound's signal to noise ratio due to background noise present in the environment and signal strength attenuation inherent in the use of external detectors to receive sound signals. Lastly, the characteristics of the measured sound (accoustic signal) signal are only indirectly related to changes in ICP and thus may give rise to inaccurate results.

Pratt, U.S. Patent No. 4,361,154 - describes a method of determining bone strength by measuring the relative speed of travel of sound through the bone. The method has particular application in determining the bone strength of a race horse's legs. The method is based on the principle that microcrushing and microfracturing which occur in bone over time in the process of absorbing shock results in a decrease in sound velocity measured across a section of the bone. Since the elastic modulus of bone is known to decrease as it weakens, there is a relationship between bone strength and rate of travel of acoustic energy through the bone. This patent, however, does not appreciate the measurement of ICP.

Rosenfeld, et al., U.S. Patent No. 4,564,022 - involves a method of non-invasively estimating ICP whereby electrical brain activity is generated by a stimulus. In particular, the patient's observance of a flashing light results in visual stimulation which causes visual evoked potentials signalled from the brain. These visual evoked potentials are measurable and their characteristics are well-defined in both children and adults. An accurate estimate of the subject's ICP can be made by measuring the latency of the second negative-going wave of the visual evoked potential. The method used to arrive at this estimate, however, is based upon the measurement of characteristics which are secondarily related to ICP. This may give results which are less accurate than those which may be obtained by measuring characteristics which are primarily related to ICP, such as vibration frequency characteristics.

Cosman, U.S. Patent No. 4,676,255 - this disclosure is a continuation-in-part of a patent relating to measuring ICP by using known pressure applied to the scalp over an implanted sensor. This particular patent focuses on measurement of negative ICP. Unfortunately, the problems which are inherent in the use of implanted (invasive) sensors are also present in this system.

Sackner, U.S. Patent No. 4,860,766 - provides a non-invasive method of monitoring intrapleural pressure of a newborn. The method is based on the principle that the cranial bones of a newborn move relative to each other during respiration as a result of a pressure wave transmitted from the pleural space through the cerebrospinal fluid and veins to the cranial cavity. The movements of the cranial bones are detected and monitored and the waveform produced directly relates to intrapleural pressure. This patent, however, does not offer any teaching that would allow one skilled in the art to measure ICP.

S.U. Patent No. 1058-556-A - this Soviet patent describes a non-invasive method of measuring ICP whereby an ultrasound sensor is positioned on one side of the front of the cranium and a pulsing signal is sent through the cranium to the occipital wall of the skull and back. The reflected ultrasound signal is recorded and ICP is determined thereby based on a set of formulas relating to amplitude of echopulsation within the cranium. This method uses sound and, therefore, the problems present in the measurement of ICP using sound (for example, a poor signal to noise ratio) are also inherent in this method.

Devine, III, et al., IBM Technical Disclosure Bulletin -describes the measurement of internal pressure in a closed chamber within a living body whereby externally applied mechanical vibration is used to induce a differential Doppler by which internal pressure can be determined. The method is geared to the measurement of ventricular pressure in the heart. The frequency, amplitude and phase of the induced vibration are known, and the reflected ultrasonic energy is detected by a receiver. This technique uses sound detection and therefore in no way overcomes the difficulties, detailed previously, associated with the use of sound to measure pressure.

Kasuga, et al., "Transmission Characteristics of Pulse Waves in the Intracranial Cavity of Dogs," Journal of Neurosurgery, Vol. 66, June 1987, pp. 907-914 -

discusses an attempt to mathematically model the intracranial pressure pulse wave transmission transfer function using the common carotid artery (randomized by using a cardiac pace-maker) as an input signal and the epidural pressure pulse wave as the output signal. The transfer function was estimated numerically from the autocorrelation function of the input signal and the cross-correlation function of the input and output signals by the least squares method. The results suggested that the lower frequencies of the pulse wave were suppressed during transmission through the intracranial cavity and that resonance was evident in the intracranial cavity under normal conditions. ICP could then be calculated using the transfer function and a known input signal. The technique used, however, has significant drawbacks. The greatest drawback is that the technique is invasive and thus involves the risk of intracranial infection. Furthermore, the technique is difficult to use, requires surgery, involves the dangers inherent in use of cardiac pacemakers, and presently is difficult to use in a clinical environment.

Kosteljanetz, et al., "Clinical Evaluation of a Simple Epidural Pressure Sensor," Acta Neurochirurgica, Vol. 83, 1986, pp. 108-111 -

This reference discusses the evaluation of Plastimed$^R$ epidural pressure (EPD) sensor in a number of patients suffering from head injury. The EPD sensor, a plastic cup 10 mm in diameter, was placed into a burrhole and two plastic tubes, one longer than the other, were connected to the cup. A pressure transducer was connected to the longer of the two tubes and the shorter tube was connected to saline reservoir via a stopcock. A conventional intraventricular pressure sensor (IVP) was situated next to the EPD sensor cup and recorded pressure continuously. The ICP values obtained from the IVP sensor were compared to those obtained from the EPD sensor. The comparison indicated that the EPD sensor gave inaccurate ICP readings. In addition to this drawback, the EPD system is invasive and requires careful alignment of the cup to the burrhole in order to obtain reasonable results. Furthermore, this method is subject to sudden sensor failure.

Takizawa, et al., "Spectral Analysis of the CSF Pulse Wave at Different Locations in the Craniospinal Axis," Journal of Neurology, Neurosurgery, and Psychiatry, Vol. 49, 1986, pp. 1135-1141 -

discusses a study intended to determine the change in frequency spectrum of the cerebrospinal fluid (CSF) pulse waveform, the amplitude transfer function from blood pressure to the CSF pulse and the conduction of each component of the CSF pulse through the CSF space under normal and abnormal conditions produced by saline infusion into the CSF space (thus elevating the pressure within the CSF space). Pressure transducers were positioned halfway between the tip of the dorsal spine and the sternum. CSF pulse and blood pressure under normal and artificially high CSF pressures were recorded at various sites. Several drawbacks are present in this method of pressure measurement. The method is invasive. Furthermore, the use of blood pressure as the input signal, with its discontinuous pressure values in between heart beats, can result in errors in the calculation of fluid pressure, since the transfer function obtained will be discrete rather than continuous.

Semmlow and Fisher, "A Noninvasive Approach To Intracranial Pressure Monitoring," Journal of Clinical Engineering, Vol. 7, March 1982, pp. 73-78 -

This reference discusses measurement of ICP by measuring the acoustical transmission properties of a skull under pressure. An impulse-like stimulus was applied to skulls suffering from various levels of elevated ICP. A piezoelectric acoustic pickup was used to monitor the acoustic response, resulting from the impulse stimulus, transmitted through the skull. Based on this monitored acoustic response, a second-order system response was modelled. Damping factor, a characteristic of a second-order system, was found to be indicative of elevated ICP. This method of ICP measurement, however, is subject to several problems. Acoustic transmission measurements, as previously mentioned, have poor signal to noise ratios. Furthermore, ICP measurements are derived, using this method, not from direct measurements but rather from artificial and often-times inaccurate mathematical system model characteristics. This may lead to largely inaccurate ICP measurements.

Accordingly, the present invention generally has as its objective the provision of both a method and an apparatus for the measurement of ICP which overcomes the above-mentioned problems in the prior art. More specifically, the present invention has as an objective the provision of a method and an apparatus for the measurement of ICP which eliminates the risks of cerebral infections.

It is a further objective of the present invention to provide a method and an apparatus for the measurement of ICP which is not subject to large measurement errors due to poor signal to noise ratios of the measured signal.

It is a further objective of the present invention to provide a method and an apparatus that measure characteristics primarily related to ICP.


SUMMARY OF THE INVENTION

The method of the present invention measures changes in the ICP of a human being or animal non-invasively (without penetrating the skull).

The method is based on the principal of physics which states that the dynamic vibration characteristics and behavior, natural frequency, mechanical impedance, coherence characteristics, and frequency response spectrum of a material (bone in this case) will change in relation to the stress applied to the elastic material.

ICP generates within the patient's skull a stress in the form an internal pressure directed to the skull bone. As the ICP varies so does the stress in the skull bone which behaves as a curved elastic plate.

It has been found, therefore, that the changes in natural frequency and frequency response spectrum of the skull bone are indicative of changes in the stress acting on the skull bone and thus, indicative of changes in ICP. These changes in natural frequency and frequency response spectrum can be measured by application of a mechanical forced oscillation stimulus (such as a vibration exciter transducer or impact hammer) which creates a mechanical wave transmission through the bone material non-invasively. The frequency response spectrum is detected by a sensor (such as an accelerometer, velocity sensor, or displacement sensor) and is analyzed and compared to the exciting spectrum by an analyzer, such as a spectrum analyzer, a dynamic signal analyzer, or network analyzer. Thus, by comparing the spectral response data obtained from the application of a given stimulus at different times, the present invention allows one to recognize ICP trends over time. In several embodiments of the present invention, the stimulus is generated and applied and the response is detected by the same device and at the same location on a patient's skull; by an impact exciter transducer, for example. However, other embodiments of present invention, generate and apply the input signal at one location on a patient's skull and measure the resultant output vibration at different place on a patient's skull. By measuring the frequency response spectrum at a point on the skull which is not effected by ICP (at locations such as, the temporal, sphenoid, lower portions of the lower occipital, lower parietal, or lower frontal bones), a baseline measurement or normal ICP pressure measurement can be obtained. This baseline measurement may then be used to measure changes in ICP from what may be considered to be a patient's normal ICP. Baseline ICP may also be obtained by measuring the frequency response spectrum of a normal patient (one without elevated ICP).

As can be readily seen, the method of the present invention offers significant advantages over the prior art. First, non-invasive measurement of ICP eliminates the risk of cerebral infections inherent in invasive methods. Furthermore, the present invention measures the characteristics of mechanical vibrations and not sound. This results in a much more favorable signal to noise ratio than in the prior art acoustic methods and therefore, more accurate results. Additionally, the present invention measures characteristics directly associated with ICP and not characteristics which are only secondarily related to ICP. This also increases the accuracy of pressure measurement results.


BRIEF DESCRIPTION OF THE DRAWINGS

The basic ICP measurement system is shown in the attached drawing wherein:

Figure 1 is a Mechanical Schematic block diagram of a human skull showing the general position of exciter and sensing transducers and a generator and analyzing apparatus;

Figure 2 is an actual reading of output gain (dB) from the analyzing apparatus plotted against output frequency components (Hz) at a series of different pressures; the skull's various frequency harmonics are readily discernible;

Figure 3 is a series of charts of output gain (dB) versus output frequency components in another series of pressures and a different series of runs; shifts in the skull's frequency harmonics are shown over the various ICP's;

Figure 4 is a plot of the figure 2 data showing a change in sensory output gain (dB) at 3.3 kHz as a function of intraskull pressure;

Figure 5 is a plot of resonance frequency (Hz) versus ICP (mm $H_2O$); this plot clearly showing that resonance frequency shifts as a function of ICP.

Figure 6 is the sensory output gain (dB) in the 200 to 300 Hz range as a function of intraskull pressure. Figure 5 and 6 being plotted from the data shown on figure 3.

Figure 7 is a block diagram of the laboratory setup used in Example 1.

## DETAILED DESCRIPTION OF THE INVENTION

### DISCUSSION OF UNDERLYING THEORY

The skull can be considered as an elastic plate in the form of a sphere, and therefore, the vibration pattern of the skull approximates the vibration pattern of a spherical elastic shell. (Reference 1. von Bekesy, G.:J. Acoust. Soc. Amer., 20:749 (1948)(Reference 2. Franks, E.K.:USAF, WADC Tech. pept. 54-24, 1954)(Reference 3. Harris, Cyril: "Shock & Vibration Handbook", ch 44, pg 17, 3rd Ed., McGraw Hill, 1988). The calculated value of elasticity of the skull, from the observed resonances, is 1.4x10p10 dynes/cm2. The Fundamental resonant frequency for the skull is between 300 and 400 Hz and resonances for the higher modes around 600 to 900 Hz. Because of its elasticity, it has been found that changes in the natural frequency and the output frequency response spectrum of the skull bone under a pressure load can be measured in the following way.

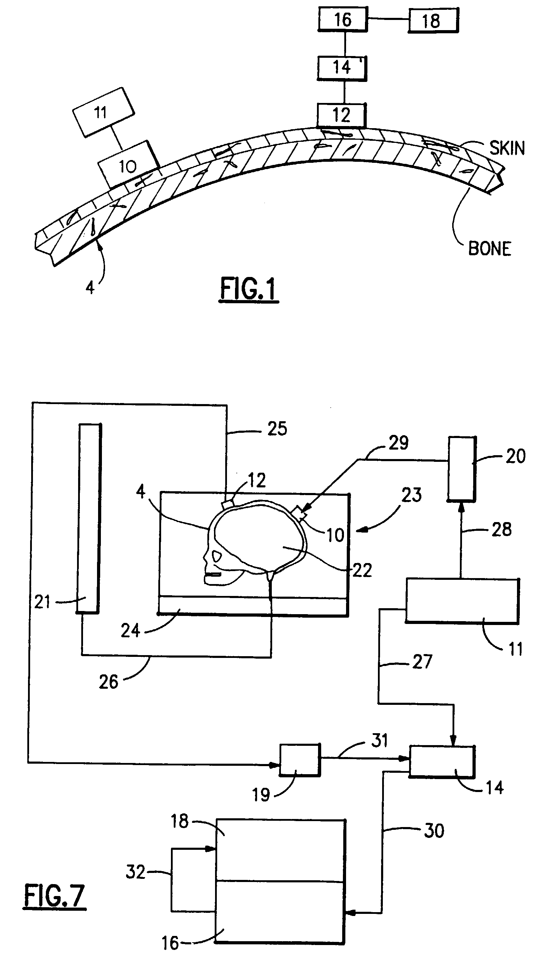

Turning to Figure 1, a mechanical exciter transducer or inpact hammer (10) is placed in contact with the outside of the skull (4) non-invasively. The exciter transducer creates a mechanical vibratory wave and/or shock wave transmission laterally through the bone material. This input vibratory and/or shock wave signal (hereinafter referred to as an exciting stimulus) applied to the skull may take the form of time varying sinusoidal, periodic, phase coherent, complex, shock, or random functions. (Reference 5. Harris, Cyril: "Shock & Vibration Handbook", ch 22, pg 4. 3rd Ed., McGraw Hill, 1988). Moreover, the input signal may also take the form of continuous sum sinusoids, broad band random, or narrow band random pattern functions. (Reference 6. Harris, Cyril: "Shock & Vibration Handbook", ch 22, pg 2. 3rd Ed., McGraw Hill, 1988) Special types of shock input signals may also be used, such as, impulse, step, half sine, decaying sinusoid, and complex functions. (Reference 7. Harris, Cyril: "Shock & Vibration Handbook", ch 23, pg 2:ch 32, pg 6. 3rd Ed., McGraw Hill, 1988)

With the application of an exciting stimulus to the skull, analysis of the frequency response spectrum sensed by the detecting transducer (12) is made. One such analysis technique is the Fourier spectrum description of the shock and/or vibration stimulus acting through a transmission medium. The Fourier analysis may be applied to a linear system when properties of a structure on which a shock or vibration act may be modelled as a function of frequency. Such properties are mathematically modelled by the transfer function in which important characteristics of the medium, through which the exciting stimulus acts, may include, mechanical impedance, mobility, transmissibility.

The Fourier analysis, therefore, may be applied in evaluating the effect of a load upon a shock or vibration source. A source of shock generally consists of a means of shock excitation and a resilient structure through which the excitation is transmitted to the skull. The character of the shock or vibration transferred through the medium is influenced by the nature of the load being driven. The characteristics of the source and load may be defined in terms of mechanical impedance or mobility.

Periodic vibration functions represented by Fourier transform series consist of a sum of sine waves whose frequencies are all multiples of a fundamental harmonic frequency; furthermore, each of the terms have varying amplitude coefficients and phase angles.

The amplitude and phase data are plotted as a frequency domain-plot known as line spectrum or a discrete frequency spectrum (vertical lines) or power spectral density (vertical line peaks connected). The resulting plots directly indicate shifts in frequency spectral response due to changes in ICP. Therefore, trends in ICP over time may be observed using the present invention.

In order to obtain a baseline or normal ICP value, a further understanding of the theory underlying the present invention is necessary. (Of course, the following discussion is only meant to show some of the methods that have been found to measure baseline or normal ICP values and in no way is meant to limit the means and/or methods available to find such values. Other variations and response characteristics may be used to find such values.)

A skull bone which is exposed to changes in load or ICP may be modelled by a circular plate with a fixed circumferential edge. This theoretical modelling gives rise to the following mathematical expression for the change in natural frequency of a circular plate with a built in circumferential edge supporting a variable load at the center, which is: (vibr Hnbk pg 42-24)

$$ Wn = \frac{\frac{t}{R^2}\sqrt{\frac{E}{\mu}}}{\sqrt{\frac{1}{N^2_p} + \frac{\pi}{n_p}\frac{M_L}{\mu t(\pi R^2)}}} $$

Where <u>Constants</u>

t = thickness of skull bone

R = radius of plate

E = Youngs modulus (lb/in$^2$)

$\mu$ = mass density of skull bone (lb-sec$^2$/in)

$n_p$ = constant based on material Poissons ratio

$N_p$ = constant based on mechanical constraint on perimeter of plate

$M_L$ = concentrated load at plate center which represents ICP.

The constants t and R are constant for a given patient. They will vary however from patient to patient. The radius of the plate, representing the skull, is measurable with a caliper or ruler.

The thickness of the plate (skull bone) is measurable by CAT (computer aided tomography) scan. Young's modulus is obtainable through demographic studies. The fundamental harmonic frequency, Wn, may be obtained experimentally for a given subject by observing the Fourier output frequency response spectrum for a given patient in a normal condition (that is, not exhibiting symptoms of abnormal ICP). The above equation may then be solved to obtain a given patient's normal ICP.

Two other methods for obtaining a normal or zero pressure ICP baseline measurement for a specific patient are presently described. For a patient with a pre-existing condition, as Hydrocephalus, periodic ICP measurements would be taken throughout the patient's past during conditions of normal pressure to establish a baseline analysis for reference should an emergency condition arise.

A baseline normal pressure could also be established by taking a spectral response measurement at a skull location not affected by ICP. One such location is at the temporal bone.

DISCUSSION OF PREFERRED EMBODIMENT

Referring now to the included drawings, there is shown in Figures 1 and 7, an exciter transducer (10) which is preferrably a Bruel and Kjaar Model 4810 vibration exciter. The exciting transducer (10) is preferably energized by a signal generator (11) such as a Bruel and Kjaar Model 1049 sine/noise generator. This input signal is also sent to a signal analyzer (14) which is preferably a spectrum analyzer, such as a Hewlett-Packard Model HP 3562A Low Frequency Spectrum Analyzer. The signal induced into a patient's skull (4) is transmitted as a wave along the skull to a sensing transducer (12) which is preferrably a Bruel and Kjaar accelerometer Model 4384. The received signal is then fed to a signal analyzer (14). The analyzed signal is then sent to a digital computer (16) such as a 80386 IBM PC-compatible computer having a MATLAB™ program for further processing the signal to provide a resultant output which can be related to ICP by change from an established norm. The analyzed signal is displayed on a computer monitor (18) to give an indication of changes in ICP or to show frequency distribution which can be related to changes in ICP.

EXAMPLE I

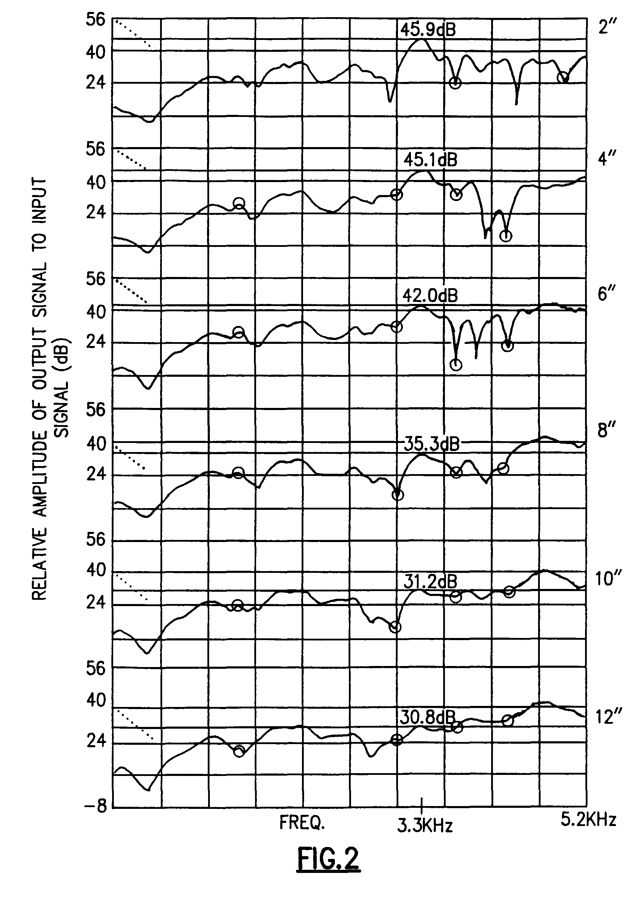

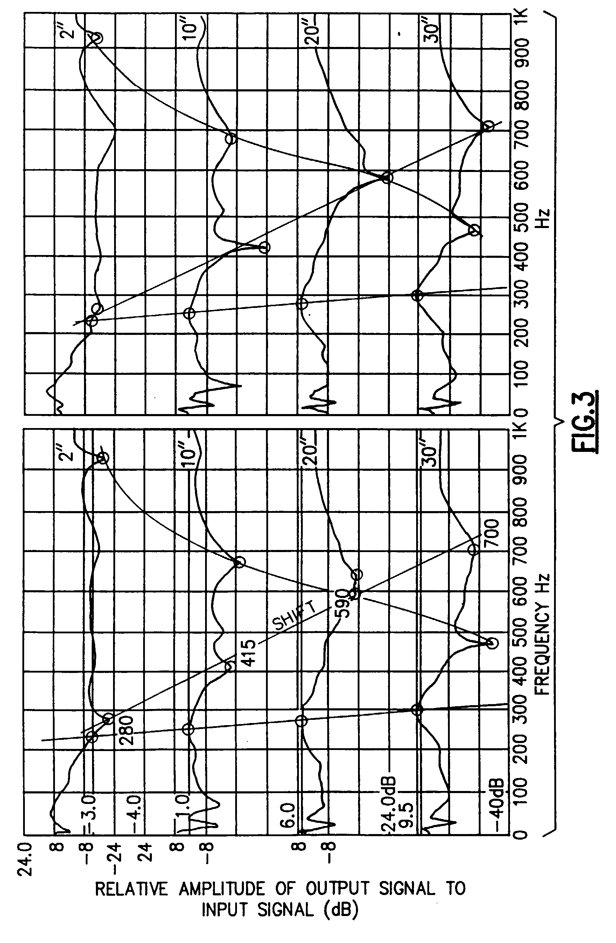

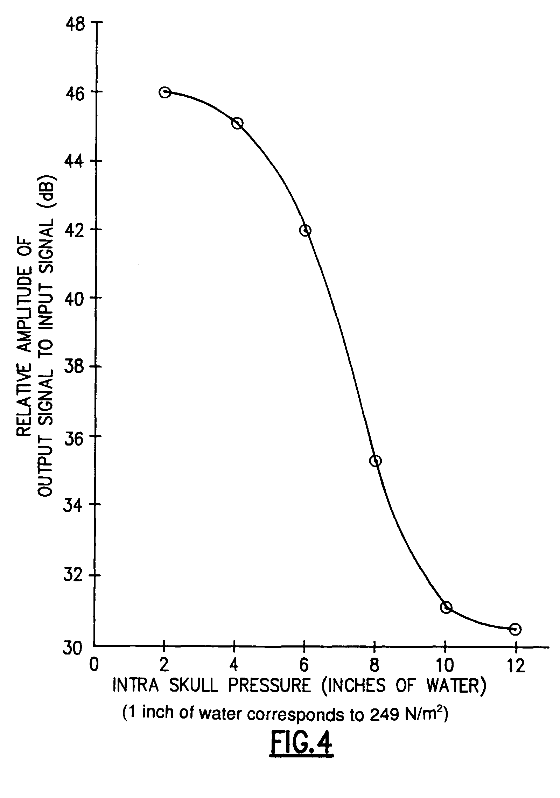

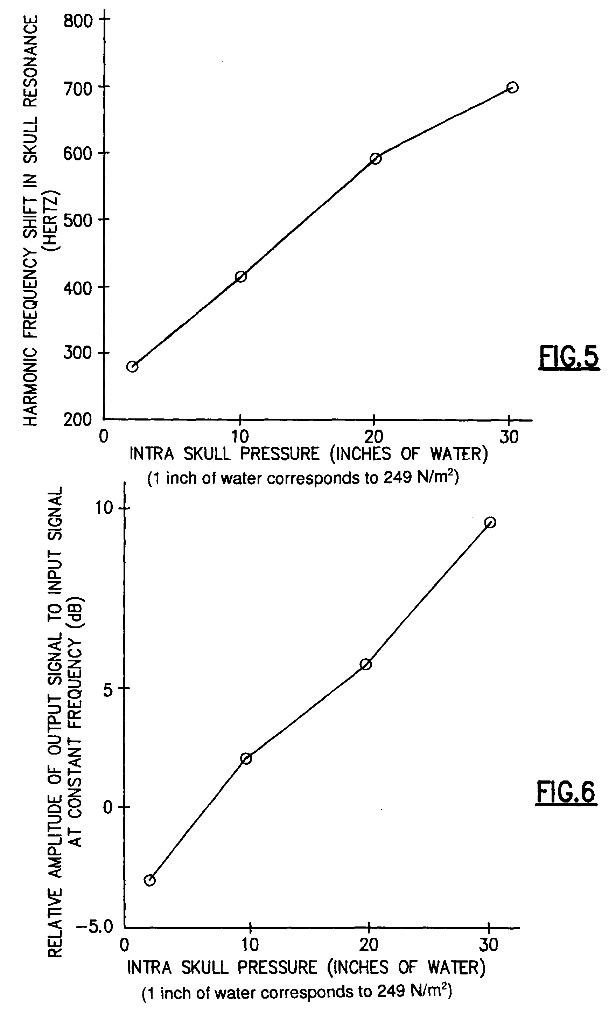

In order to test the theoretical basis of the invention as discussed above, a skull (4) was employed which contained a bladder (22) into which water of a predetermined and controlled pressure could be injected from a water column (21) via a tube (26). The skull (4) was placed into a containment box (23) in which was placed a shock-absorbing cushion (24) to isolate the skull (4) from ambient background vibrations. The amount of water injected into the skull (4) was recorded for each run and ICP derived therefrom. A signal was induced in the skull by placing the transducer (10) in contact with the skull and activating the transducer (10) by setting the signal generator (11) in an autocorrelation mode and relaying this signal through a cable (28) to an amplifier which was set at a gain of 10 (20) and finally through another cable (29) to the transducer (10). The resultant vibratory wave induced in the skull (4) was received by the sensing transducer (12) and relayed via a cable (25) to an amplifier (19). This amplifier then relayed the signal through a cable (31) to the signal analyzer (14). The analyzer then performed computations on the input signal and the output signal and a signal indicative of these computations was then sent to a digital computer (16) through cable (30). Various algorithms were employed by the digital computer (16) to correlate the raw data and after being transmitted through a cable (32) to a screen (18) the correlated raw data was stored. The raw data obtained from the system is plotted in figures 2 and 3 at various runs of different pressures. Additionally, the decibel output as a function of frequency for each of the number of pressures of the liquid within the skull (4) is plotted in figures 2 and 3. Selected points on the various series of curves were then plotted in figures 4, 5, and 6 to show a change in measured characteristic either intensity or harmonic frequency shift as a function of pressure, thus demonstrating the ability of the device to clearly correlate a change in a laboratory response of the simulated skull as a function of the liquid pressure within the skull.

While one preferred assembly of standard components has been described above numerous modifications thereof are possible.

## APPENDIX A

This appendix gives an overview of the most important technical characteristics of the equipment used in Example 1.

### Sensing Transducer

Bruel & Kjaar Model 4384 Piezoelectric Accelerometer:

|  |  |  |
|---|---|---|
| Voltage Sensitivity | about | 0.8 mV-s$^2$/m |
| Frequency Range | 5% | 0.2 - 9,100 Hz |
|  | 10% | 0.1 - 12,600 Hz |
| Capacitance |  | 1200 pF |
| Typical Acoustic Sensitivity |  | 0.01 m/s$^2$ |
| Maximum Operational Shock |  | 200 km/s$^2$ |
| Maximum Operational Continuous Sinusoidal Accel. Peak |  | 60 km/s$^2$ |

### Exciting Transducer

Bruel & Kjaar Model 4810 Small Vibration Exciter:

| | |
|---|---|
| Force Rating Sine Peak | 10 N |
| Max. Bare Table Accel. Peak | 500 m/s$^2$ |
| Max. Displacement P-P | 6 mm |
| First Resonance Freq. | 18 kHz |

### Signal Generator

Bruel & Kjaar Model 1049 Sine/Noise Generator:

Output Modes

| | |
|---|---|
| Sine: | 0.2 Hz - 200 kHz |
| Narrow Band Noise: | BW 1 to 316 Hz |
| White Noise: | 9 Freq. Ranges |
| Pink Noise: | 9 Freq. Ranges |
| Max. Output Voltage (Current) | 5 V (100 mA) |

Distortion

Sine

| | |
|---|---|
| 0.2 Hz - 100 kHz | less than -85 dB |
| 100 kHz - 200 kHz | less than -75 dB |

Random

| | |
|---|---|
| 0.2 Hz - 100 kHz | less than -73 dB |
| 100 kHz - 200 kHz | less than -63 dB |

### Signal Analyzer

Hewlett Packard Model HP 3562A Low Frequency Spectrum Analyzer:

The reader is directed to Hewlett Packard's technical specification for this device.

## EP 0 541 671 B1

<u>Spectrum Analysis Software</u>

Mathworks MATLAB$^{TM}$ Signal Processing Toolbox:

This is an optional extension module designed to be used with MATLAB$^{TM}$. It offers application-specific capabilities in the area of digital signal processing and time-series analysis. Central features of Signal Processing Toolbox are functions that implement the most useful digital filtering and power spectrum estimation (FFT) techniques. Discrete Fourier transforms and other related spectral transformations may be calculated with this package. Furthermore, estimation of the power spectra of signals, detection of narrow-band signals buried in wide-band noise, calculation of power spectral density, cross spectral density, transfer function characteristics, and coherence functions are also possible with this software.

## Claims

1. A method of non-invasively measuring changes in intracranial pressure in a patient's skull comprising the steps of:

   a) generating a predetermined input mechanical vibration signal and applying said input signal to a first location on a patient's skull;
   b) detecting an output mechanical vibration signal from a second location on a patient's skull by means of an accelerometer (12), said output signal being a modification of said input signal, said input signal having been changed as a function of intracranial pressure as it travels from said first location to said second location;
   c) storing data characteristic of said input and output signals into a data base; and
   d) repeating steps (a), (b), and (c) and comparing said data stored in said data base to detect changes in intracranial pressure,
   characterised in that changes in intracranial pressure are detected by monitoring the harmonic frequency shift of said output mechanical vibration signal.

2. A method according to claim 1, characterised in that the input signal comprises a shock signal.

3. A method according to claim 1 or 2 characterised in that

   a) said first location and said second location are located at substantially the same place on said patient's skull; and/or
   b) the means for generating comprises a vibration exciter transducer.

4. A method according to any one of claims 1 to 3 characterised in that an input mechanical vibration signal is applied and an output mechanical vibration signal is detected at points which are not affected by variations in intracranial pressure, said output mechanical vibration signal providing harmonic frequency data against which harmonic frequency shifts with varying intracranial pressure may be compared.

**5.** A method according to claim 4, characterised in that the input and output mechanical vibrations are applied and detected at substantially the same place on said patient's skull.

**6.** A method according to any one of claims 1 to 5 of measuring a patient's intracranial pressure from said patient's normal intracranial pressure, characterised by measuring the thickness of a patient's skull non-invasively by using non-invasive thickness measuring means and estimating said patient's normal intracranial pressure, by means of a vibration signal, from said data by using estimation means.

**7.** An apparatus for non-invasively measuring changes in intracranial pressure in a patient's skull comprising:

a) means (10) for generating a predetermined input mechanical vibration signal and applying said signal to a first location on a patient's skull;
b) an accelerometer (12) for detecting an output mechanical vibration signal from a second location on the patient's skull, said output signal being a modification of said input signal, said input signal having been changed as a function of intracranial pressure as it travels from said first location to said second location; and
c) means (16) for storing data characteristic of said input and output signals into a data base; characterised by
d) means (14, 16) for comparing data stored in said data base from a plurality of input and output signals so as to detect shifts in harmonic frequency due to changes in intracranial pressure.

**8.** An apparatus according to claim 7, characterised in that

a) the means (10) for generating and applying said input signal comprises an impact hammer or a mechanical vibration exciter transducer.

**9.** An apparatus according to claim 7 or 8, characterised in that:

the means (10) for generating and applying said input signal, and the accelerometer (12) for detecting said output signal comprise a mechanical vibration exciter transducer configured so that said first location and second location are located at substantially the same location on said patient's skull.

**10.** An apparatus according to claim 7, 8 or 9 for measuring changes in a patient's intracranial pressure from said patient's normal intracranial pressure, characterised by:

a) means (10) for generating a predetermined input mechanical vibration signal and applying this input signal at a third location on a patient's skull which is not affected by changes in intracranial pressure;
b) means (12) for detecting an output mechanical vibration signal from a fourth location on said patient's skull which is not affected by changes in intracranial pressure, said output signal being a modification of said input signal, said input signal having been changed as a function of said patient's normal intracranial pressure;
c) means (16) for storing harmonic frequency data characteristic of the input signal applied at the third location and the output signal detected at the fourth location into the data base;
d) means (14, 16) for comparing harmonic frequency data characteristics of the input signal applied at the first location and the output signal detected at the second location to harmonic frequency data characteristic of the input signal applied at the third location and the output signal detected at the fourth location.

**11.** An apparatus according to claim 10, characterised in that said third location and said fourth location are located at substantially the same place on said patient's skull.

**12.** An apparatus according to any one of claims 7 to 11 for measuring changes in a patient's intracranial pressure from said patient's normal intracranial pressure, characterised by:

a) means for non-invasively measuring the thickness of a patient's skull; and
b) means for estimating said patient's normal intracranial pressure, by means of a vibration signal, from said data by using estimation means.

**Patentansprüche**

**1.** Verfahren zur nicht-invasiven Messung von Änderungen des intrakranialen Drucks in dem Schädel eines Patienten mit folgenden Schritten:

a) Erzeugen eines vorbestimmten Eingangssignals einer mechanischen Schwingung und Anlegen des Eingangssignals an eine erste Stelle an dem Schädel eines Patienten;

b) Detektieren eines Ausgangssignals einer mechanischen Schwingung von einer zweiten Stelle an dem Schädel eines Patienten mit einem Beschleunigungsmesser (12), wobei das Ausgangssignal eine Modifikation des Eingangssignals ist, wobei sich das Eingangssignal als eine Funktion des intrakranialen Drucks bei der Fortpflanzung von der ersten Stelle zu der zweiten Stelle geändert hat;

c) Speichern der Datencharakteristik des Eingangssignals und des Ausgangssignals in einer Datenbank, und

d) Wiederholen der Schritte a), b) und c) und Vergleichen der in der Datenbank gespeicherten Daten, um Änderungen des intrakranialen Drucks festzustellen,
dadurch gekennzeichnet, daß Änderungen des intrakranialen Drucks durch Überwachen der Verschiebung der harmonischen Frequenz des Ausgangssignals einer mechanischen Schwingung festgestellt werden.

2. Verfahren nach Anspruch 1, dadurch gekennzeichnet, daß das Eingangssignals ein Stoßsignal umfaßt.

3. Verfahren nach Anspruch 1 oder 2, dadurch gekennzeichnet, daß

a) die erste Stelle und die zweite Stelle im wesentlichen an dem gleichen Ort an dem Schädel des Patienten liegen, und/oder

b) die Vorrichtung zum Erzeugen einen Wandler für die Schwingungserregung umfaßt.

4. Verfahren nach einem der Ansprüche 1 bis 3, dadurch gekennzeichnet, daß ein Eingangssignal einer mechanischen Schwingung angelegt wird, und ein Ausgangssignal einer mechanischer Schwingung an Punkten detektiert wird, die nicht durch Änderungen des intrakranialen Drucks beeinflußt werden, wobei das Ausgangssignal einer mechanischen Schwingung Daten harmonischer Frequenzen zur Verfügung stellt, mit denen Verschiebungen harmonischer Frequenzen bei sich veränderndem intrakranialen Druck verglichen werden können.

5. Verfahren nach Anspruch 4, dadurch gekennzeichnet, daß mechanische Eingangsschwingungen und Ausgangsschwingungen angelegt und an im wesentlichen dem gleichen Ort an dem Schädel des Patienten detektiert werden.

6. Verfahren nach einem der Ansprüche 1 bis 5 zur Messung eines intrakranialen Drucks des Patienten aus dem normalen intrakranialen Druck des Patienten, gekennzeichnet durch Messen der Dicke eines dem Schädel eines Patienten auf nicht-invasive Weise durch Einsatz einer nicht-invasiven Dickenmeßvorrichtung und Abschätzen des normalen intrakranialen Drucks des Patienten mit Hilfe eines Schwingungssignals aus den Daten unter Einsatz einer Abschätzvorrichtung.

7. Vorrichtung zum nicht-invasiven Messen von Änderungen des intrakranialen Drucks in dem Schädel eines Patienten mit

a) einer Vorrichtung (10) zum Erzeugen eines vorbestimmten Eingangssignals einer mechanischen Schwingung und Anlegen des Signals an einer ersten Stelle an dem Schädel eines Patienten;

b) einem Beschleunigungsmesser (12) zum Detektieren eines Ausgangssignals einer mechanischen Schwingung an einer zweiten Stelle an dem Schädel des Patienten, wobei das Ausgangssignal eine Modifikation des Eingangssignals ist, wobei sich das Eingangssignal als eine Funktion des intrakranialen Drucks bei der Fortpflanzung von der ersten Stelle zu der zweiten Stelle geändert hat; und

c) einer Vorrichtung (16) zum Speichern der Datencharakteristik des Eingangs- und des Ausgangssignals in einer Datenbank, gekennzeichnet durch

d) eine Vorrichtung (14, 16) zum Vergleichen der in der Datenbank gespeicherten Daten einer Vielzahl von Eingangs- und Ausgangssignalen, um Verschiebungen der harmonischen Frequenz in Folge von Änderungen des intrakranialen Drucks festzustellen.

8. Vorrichtung nach Anspruch 7, dadurch gekennzeichnet, daß

   a) die Vorrichtung (10) zum Erzeugen und Anlegen des Eingangssignals einen Stoßhammer oder einen Wandler für eine mechanische Schwingungserregung aufweist.

9. Vorrichtung nach Anspruch 7 oder 8, dadurch gekennzeichnet, daß

   die Vorrichtung (10) zum Erzeugen und Anlegen des Eingangssignals und der Beschleunigungsmesser (12) zum Detektieren des Ausgangssignals einen Wandler für eine mechanische Schwingungserregung umfassen, der derart ausgelegt ist, daß die erste Stelle und die zweite Stelle an im wesentlichen dem gleichen Ort an dem Schädel des Patienten angeordnet sind.

10. Vorrichtung nach Anspruch 7, 8 oder 9 zum Messen von Änderungen des intrakranialen Drucks eines Patienten aus dem normalen intrakranialen Druck des Patienten, gekennzeichnet durch

    a) eine Vorrichtung (10) zum Erzeugen eines vorbestimmten Eingangssignals einer mechanischen Schwingung und Anlegen dieses Eingangssignals an einer dritten Stelle an einem dem Schädel eines Patienten, die nicht durch Änderungen des intrakranialen Drucks beeinflußt wird,

    b) eine Vorrichtung (12) zum Detektieren eines Ausgangssignals einer mechanischen Schwingung an einer vierten Stelle an dem Schädel des Patienten, die nicht durch Änderungen des intrakranialen Drucks beeinflußt wird, wobei das Ausgangssignal eine Modifikation des Eingangssignals darstellt, wobei sich das Eingangssignal als Funktion des normalen intrakranialen Drucks des Patienten geändert hat;

    c) eine Vorrichtung (16) zum Speichern der Datencharakteristik der harmonischen Frequenz des Eingangssignals, das an der dritten Stelle angelegt wurde, und des Ausgangssignals in der Datenbank, das an der vierten Stelle detektiert wurde;

    d) eine Vorrichtung (14, 16) zum Vergleichen der Datencharakteristik der harmonischen Frequenz des Eingangssignals, das an die erste Stelle angelegt wurde, und des Ausgangssignals, das an der zweiten Stelle detektiert wurde, mit der Datencharakteristik der harmonischen Frequenz des Eingangssignals, das an der dritten Stelle angelegt wurde, und des Ausgangssignals, das an der vierten Stelle detektiert wurde.

11. Vorrichtung nach Anspruch 10, dadurch gekennzeichnet, daß die dritte Stelle und die vierte Stelle an im wesentlichen dem gleichen Ort an dem Schädel des Patienten angeordnet sind.

12. Vorrichtung nach einem der Ansprüche 7 bis 11 zur Messung von Änderungen des intrakranialen Drucks eines Patienten aus dem normalen intrakranialen Druck eines Patienten, gekennzeichnet durch

    a) eine Vorrichtung zum nicht-invasiven Messen der Dicke des Schädels eines Patienten, und

    b) eine Vorrichtung zum Abschätzen des normalen intrakranialen Drucks des Patienten mit Hilfe eines Schwingungssignals aus den Daten unter Verwendung einer Abschätzvorrichtung.

**Revendications**

1. Procédé de mesure non invasive des changements de pression intracrânienne dans un crâne de patient comprenant les étapes consistant à :

   a) générer un signal de vibrations mécaniques d'entrée prédéterminé et appliquer ledit signal d'entrée à un premier emplacement sur un crâne de patient,
   b) détecter un signal de vibrations mécaniques de sortie à partir d'un second emplacement sur un crâne de patient au moyen d'un accéléromètre (12), ledit signal de sortie constituant une modification dudit signai d'entrée, ledit signal d'entrée ayant été modifié en fonction de la pression intracrânienne en se propageant depuis ledit premier emplacement vers ledit second emplacement,
   c) mémoriser une caractéristique de données desdits signaux d'entrée et de sortie dans une base de données, et
   d) répéter les étapes (a), (b) et (c) et comparer lesdites données mémorisées dans ladite base de données afin de détecter des chargements de la pression intracrânienne,
caractérisé en ce que des changements de la pression intracrânienne sont détectés en surveillant le décalage de fréquences harmoniques dudit signal de vibrations mécaniques de sortie.

2. Procédé selon la revendication 1, caractérisé en ce que le signal d'entrée comprend un signal de choc.

3. Procédé selon la revendication 1 ou 2, caractérisé en ce que

   a) ledit premier emplacement et ledit second emplacement sont situés pratiquement au même endroit sur ledit crâne du patient, et/ou
   b) le moyen destiné à générer comprend un transducteur excitateur de vibrations.

4. Procédé selon l'une quelconque des revendications 1 à 3, caractérisé en ce qu'un signal de vibrations mécaniques d'entrée est appliqué et un signal de vibrations mécaniques de sortie est détecté en des points qui ne sont pas affectés par des variations de la pression intracrânienne, ledit signal de vibrations mécaniques de sortie fournissant des données de fréquences harmoniques vis-à-vis desquelles des décalages de fréquences harmoniques en fonction d'une pression intracrânienne variable, peuvent être comparés.

5. Procédé selon la revendication 4, caractérisé en ce que les vibrations mécaniques d'entrée et de sortie sont appliquées et détectées pratiquement au même endroit sur ledit crâne du patient.

6. Procédé selon l'une quelconque des revendications 1 à 5 consistant à mesurer une pression intracrânienne d'un patient par rapport à ladite pression intracrânienne normale d'un patient, caractérisé par la mesure non invasive de l'épaisseur d'un crâne de patient en utilisant un moyen de mesure d'épaisseur non invasif et en estimant ladite pression intracrânienne normale du patient, au moyen d'un signal de vibrations, à partir desdites données grâce à l'utilisation d'un moyen d'estimation.

7. Appareil destiné à la mesure non invasive des changements de pression intracrânienne dans un crâne de patient comprenant :

   a) un moyen (10) destiné à générer un signal de vibrations mécaniques d'entrée prédéterminé et appliquer ledit signal à un premier emplacement sur un crâne de patient,
   b) un accéléromètre (12) destiné à détecter un signal de vibrations mécaniques de sortie à partir d'un second emplacement sur le crâne du patient, ledit signal de sortie étant une modification dudit signal d'entrée, ledit signal d'entrée ayant été changé en fonction de la pression intracrânienne en se propageant dudit premier emplacement audit second emplacement, et
   c) un moyen (16) destiné à mémoriser une caractéristique de données desdits signaux d'entrée et de sortie dans une base de données, caractérisé par
   d) un moyen (14, 16) destiné à comparer des données mémorisées dans ladite base de données vis-à-vis d'une pluralité de signaux d'entrée et de sortie de façon à détecter des décalages de fréquences harmoniques dus à des changements de pression intracrânienne.

8. Appareil selon la revendication 7, caractérisé en ce que

   a) le moyen (10) destiné à générer et appliquer ledit signal d'entrée comprend un marteau à percussion ou un transducteur excitateur de vibrations mécaniques.

9. Appareil selon la revendication 7 ou 8, caractérisé en ce que :

   le moyen (10) destiné à générer et appliquer ledit signal d'entrée, et l'accéléromètre (12) destiné à détecter ledit signal de sortie, comprennent un transducteur excitateur de vibrations mécaniques configuré de manière à ce que ledit premier emplacement et ledit second emplacement soient situés pratiquement au même emplacement sur ledit crâne du patient.

10. Appareil selon la revendication 7, 8 ou 9 destiné à mesurer des changements d'une pression intracrânienne d'un patient par rapport à ladite pression intracrânienne normale du patient, caractérisé par

    a) un moyen (10) destiné a générer un signal de vibrations mécaniques d'entrée prédéterminé et appliquer ce

**12**

signal d'entrée à un troisième emplacement sur un crâne de patient, qui n'est pas affecté par des changements de pression intracrânienne,

b) un moyen (12) destiné à détecter un signal de vibrations mécaniques de sortie à partir d'un quatrième emplacement sur ledit crâne du patient qui n'est pas affecté par des changements de pression intracrânienne, ledit signal de sortie étant une modification dudit signal d'entrée, ledit signal d'entrée ayant été chargé en fonction de ladite pression intracrânienne normale du patient,

c) un moyen (16) destiné à mémoriser une caractéristique de données de fréquences harmoniques du signal d'entrée appliqué au troisième emplacement et du signal de sortie détecté au quatrième emplacement, dans la base de données,

d) un moyen (14, 16) destiné à comparer des caractéristiques de données de fréquences harmoniques du signal d'entrée appliqué au premier emplacement et du signal de sortie détecté au second emplacement à une caractéristique de données de fréquences harmoniques du signal d'entrée appliqué au troisième emplacement et du signal de sortie détecté au quatrième emplacement.

11. Appareil selon la revendication 10, caractérisé en ce que ledit troisième emplacement et ledit quatrième emplacement sont situés pratiquement au même endroit sur ledit crâne du patient.

12. Appareil selon l'une quelconque des revendications 7 à 11 destiné à mesurer des changements d'une pression intracrânienne d'un patient par rapport à ladite pression intracrânienne normale du patient, caractérisé par :

a) un moyen destiné à la mesure non invasive de l'épaisseur d'un crâne de patient, et

b) un moyen destiné à estimer ladite pression intracrânienne normale du patient au moyen d'un signal de vibrations, à partir desdites données en utilisant un moyen d'estimation.

FIG.1

FIG.7

**FIG.2**

FIG.3

EP 0 541 671 B1

RELATIVE AMPLITUDE OF
OUTPUT SIGNAL TO INPUT SIGNAL (dB)

INTRA SKULL PRESSURE (INCHES OF WATER)

(1 inch of water corresponds to 249 N/m²)

## FIG.4

FIG.5

FIG.6

18